Europäisches Patentamt

European Patent Office

Office européen des brevets

(11) Numéro de publication : **0 433 127 A2**

(12) # DEMANDE DE BREVET EUROPEEN

(21) Numéro de dépôt : 90403427.9

(22) Date de dépôt : 03.12.90

(51) Int. Cl.⁵ : **C08B 37/00, C12P 19/04, A61K 31/72**

(30) Priorité : 04.12.89 FR 8915972

(43) Date de publication de la demande :
19.06.91 Bulletin 91/25

(84) Etats contractants désignés :
AT BE CH DE DK ES FR GB GR IT LI LU NL SE

(71) Demandeur : ROUSSEL-UCLAF
35, boulevard des Invalides
F-75007 Paris (FR)

(72) Inventeur : Smets, Pierre
137, Avenue du Maréchal Leclerc
F-78670 Villennes sur Seine (FR)
Inventeur : Zalisz, René
20, rue Delattre de Tassigny
F-95180 Menucourt (FR)

(74) Mandataire : Vieillefosse, Jean-Claude et al
Roussel-Uclaf 111, route de Noisy B.P. 9
F-93230 Romainville (FR)

(54) Nouveau dérivé sulfaté du galactanne extrait de Klebsiella, procédé de préparation, application à titre de médicaments et compositions pharmaceutiques le renfermant.

(57) Nouveau dérivé sulfaté du galactanne extrait de Klebsiella essentiellement constitué d'oses neutres sulfatés dans une proportion de 20 à 90% des hydroxyles.
Procédé de préparation, application à titre de médicament de ce nouveau dérivé.

EP 0 433 127 A2

# NOUVEAU DERIVE SULFATE DU GALACTANNE EXTRAIT DE KLEBSIELLA, PROCEDE DE PREPARATION, APPLICATION A TITRE DE MEDICAMENTS ET COMPOSITIONS PHARMACEUTIQUES LE RENFERMANT

La présente invention concerne un nouveau dérivé sulfaté du galactanne extrait de Klebsiella, son procédé de préparation, son application à titre de médicaments et les compositions pharmaceutiques le renfermant.

Le brevet français FR 2 574 429 décrit des acylglycannes extraits de Klebsiella constitués d'environ 80% d'oses neutres, 20% de lipides, moins de 2% de protéines, ayant un poids moléculaire d'environ 12500 et renfermant un enchaînement de galactoses liés en 1-3. Ils sont doués de propriétés antiallergiques et immunomodulatrices.

La demande de brevet français n° 89.09305 déposée le 11 juillet 1989, et la demande de brevet européen n° 904019874 décrivent du galactanne à faible poids moléculaire extrait de Klebsiella, son procédé de préparation, son application à titre de médicaments, notamment de médicaments immunostimulants.

La présente invention concerne un nouveau dérivé sulfaté du galactanne extrait de Klebsiella, préparé à partir du galactanne obtenu précédemment.

L'invention a aussi pour objet un nouveau dérivé sulfaté du galactanne extrait de Klebsiella, caractérisé en ce qu'il est essentiellement constitué d'oses neutres sulfatés dans une proportion de 20 à 90% des hydroxyles.

La composition en oses neutres est déterminée par chromatographie en phase gazeuse après méthanolyse selon la méthode ZANETTA J. Chromato. (1972) 69, p. 291 ou la méthode de KAMERLING (Biochem J. (1975), 151, p. 491).

L'invention a plus particulièrement pour objet un dérivé sulfaté du galactanne caractérisé en ce qu'il est essentiellement constitué d'oses neutres sulfatés dans une proportion de 40 à 80% des hydroxyles.

Parmi les produits objet de l'invention, on retient de préférence un dérivé sulfaté du galactanne caractérisé en ce qu'il est essentiellement constitué d'oses neutres sulfatés dans une proportion de 50 à 70% des hydroxyles.

Selon l'invention, le dérivé sulfaté du galactanne est caractérisé en ce qu'il est constitué d'un enchaînement linéaire de galactose en 1-3 et a un poids moléculaire compris entre 5000 et 12000 et de préférence voisin de 7000.

Ces polysaccharides sont exempts de lipides et de protéines.

La composition en galactose est déterminée par chromatographie en phase gazeuse, après réduction et méthanolyse.

Les lipides sont dosés par chromatographie en phase gazeuse après méthanolyse.

Les protéines sont dosées par la méthode de LOWRY (J.B.C (1951), 193, p. 265-273.

La séquence d'enchaînement des galactoses est déterminée par les méthodes classiques de méthylation, analyse par chromatographie en phase gazeuse couplée à la spectrométrie de masse, oxydation per iodique, RMN de $^1$H et $^{13}$C. Les résultats montrent que le galactanne est formé d'une chaîne linéaire de résidus de galactose reliés par des liaisons alpha (1-3) 50% et béta (1-3) 50%, avec un motif de répétition constitué de 8 résidus galactopyranose et 1 résidu galactofuranose.

Le dérivé sulfaté du galactanne objet de l'invention peut être préparé à partir de différentes espèces de Klebsiella. On retient tout particulièrement le dérivé sulfaté du galactanne caractérisé en ce qu'il provient de Klebsiella pneumoniae, et notamment de la souche déposée à l'Institut Pasteur à Paris ou collection Nationale de culture de Microorganismes (CNCM) sous les numéros 52145 et 1-163 et à la National Culture Type collection sous le n° 5055.

La présente invention a également pour objet un procédé de préparation du nouveau dérivé sulfaté du galactanne extrait de Klebsiella tel que défini ci-dessus qui consiste à traiter un acylglycanne extrait de Klebsiella, essentiellement constitué d'environ 80% d'oses neutres, 20% de lipides, moins de 2% de protéines et ayant un poids moléculaire d'environ 12500, par hydrolyse acide douce, chromatographie à haute performance sur échangeur d'anions, récupère la fraction non retenue, soumet à une filtration sur gel, récupère la fraction contenant le galactanne de poids moléculaire compris entre 4000 et 10000, procédé caractérisé en ce que l'on sulfate la fraction de galactanne ainsi obtenu, purifie par dialyse et isole le dérivé sulfaté du galactanne ainsi obtenu.

L'acylglycanne extrait de Klebsiella de départ peut être obtenu à partir d'un extrait bactérien hydrosoluble de Klebsiella par chauffage puis fractionnement chromatographique. De telles préparations ont déjà été décrites dans la demande de brevet français FR 2 574 429 et le brevet allemand DE 3 543 267.

L'extrait bactérien hydrosoluble de Klebsiella a été préparé selon les procédés décrits dans les brevets FR 2 490 496 et EP 49182.

Dans des conditions préférentielles de mise en oeuvre du procédé objet de l'invention :

- L'hydrolyse acide douce de l'acylglycanne est effectuée dans une solution à 1% d'acide acétique par chauffage à 100°C pendant 90 minutes ; le précipité formé, qui renferme la fraction lipidique, est éliminé par centrifugation, par exemple 30 minutes à 2000 G. Le surnageant qui renferme le galactanne est recueilli et peut être lyophilisé.

- Le galactanne est alors séparé du surnageant par fractionnement chromatographique. On recueille la fraction constituée d'oses neutres.

- Le fractionnement peut être effectué par chromatographie sur échangeurs d'anions, de préférence par chromatographie haute performance, par exemple sur Magnum 9SAX Whatman. La chromatographie à haute performance sur échangeur d'anions consiste à séparer le galactanne par élution à l'eau. La fraction intéressante, constituée d'oses neutres, est repérée par détection spectrophotométrique à 200 nm et à 492 nm après coloration au test phénol-acide sulfurique, selon la méthode de DUBOIS (Anal. chem. (1950) 28, p. 350).

- Ladite fraction, constituée d'oses neutres, est soumise à une filtration sur gel qui permet de récupérer la fraction contenant du galactanne de poids moléculaire compris entre 4000 et 10000. La filtration sur gel est effectuée sur des supports commercialisés, par exemple sur Sephadex ou Biogel agarose ; on utilise de préférence Biogel A-1.5M.

- La sulfatation de la fraction de galactanne est effectuée au moyen d'un acide sulfonique tel que l'acide chlorosulfonique au sein d'une amine telle que la pyridine ou au moyen d'un sulfonate tel que le N-sulfonate de pipéridine en opérant par chauffage du milieu réactionnel.

La dialyse est effectuée contre de l'eau distillée.

L'invention a également pour objet le dérivé sulfaté du galactanne tel qu'obtenu lors de la mise en oeuvre du procédé décrit ci-dessus.

Le dérivé sulfaté du galactanne, objet de la présente invention, possède de très intéressantes propriétés pharmacologiques ; il est doué notamment de remarquables propriétés immunostimulantes ainsi que d'une bonne tolérance. Il possède notamment la propriété de stimuler la production d'Il$_1$ et de TNF au niveau des macrophages et surtout de stimuler la génération de radicaux libres (polynucléaires). Il permet également de synergiser l'effet du GM-CSF (Granulomonocyte-Colony Stimulating Factor).

On note également une remarquable activité anti-élastase, tant contre l'élastase pancréatique bovine, que contre l'élastase leucocytaire humaine. Ces produits présentent également une activité anti-coagulante.

Ces propriétés sont illustrées plus loin dans la partie expérimentale.

Ces propriétés justifient l'utilisation du dérivé sulfaté du galactanne tel que défini ci-dessus à titre de médicaments.

Ces médicaments trouvent, par exemple, leur emploi dans le traitement ou la prévention chez l'homme des immunodépressions, des maladies infectieuses causées par les bactéries ou les virus, et en particulier par le virus du Sida dans le traitement des maladies à parasites, des toxi-infections, dans le traitement des infections posthospitalières et postchirurgicales et des allergies de toutes origines. Ces médicaments peuvent également être utilisés très avantageusement dans le traitement des greffes de moelle osseuse et des aplasies médullaires postchimiothérapiques.

La dose usuelle, variable selon le produit utilisé, le sujet traité et l'affection en cause, peut être par exemple de 0,5 à 5 mg par jour par voie orale.

Ces médicaments trouvent également, par exemple, leur emploi en pneumologie dans le traitement de l'emphysème, des pneumonies, des bronchites, des troubles pulmonaires causés par le tabagisme ou la pollution atmosphérique, en cardiologie dans le traitement des arthrites, ainsi que par exemple en dermatologie dans le traitement du psoriasis, des brûlures, des buloses et dans le vieillissement de la peau, en gastroentérologie, dans le traitement des pancréatites aigues et d'une manière générale dans le traitement de toutes les affections mettant en cause le disfonctionnement de l'élastase ainsi que dans le traitement de la maladie thrombo-embolique.

La dose usuelle, variable selon le produit utilisé, le sujet traité et l'affection en cause peut être par exemple de 1 à 300 mg par jour par voie intraveineuse chez l'homme.

L'invention a également pour objet les compositions pharmaceutiques qui renferment le dérivé sulfaté du galactanne extrait de Klebsiella tel que défini ci-dessus à titre de principe actif.

A titre de médicaments, le dérivé sulfaté du galactanne extrait de Klebsiella tel que défini ci-dessus, peut être incorporé dans des compositions pharmaceutiques destinées à la voie digestive, parentérale ou locale.

Les compositions pharmaceutiques correspondantes peuvent être, par exemple, solides ou liquides et se présenter sous les formes pharmaceutiques couramment utilisées en médecine humaine, comme par exemple, les comprimés, simples ou dragéifiés, les gélules, les granulés, les solutions, les sirops, les suppositoires, les préparations injectables lyophilisées ou non, les ovules, les crèmes, les pommades, les lotions, les gouttes,

3

les collyres, les aérosols ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Il va être donné maintenant à titre non limitatif, des exemples de mise en oeuvre de l'invention.

### EXEMPLE 1 : Dérivé sulfaté du galactanne extrait de Klebsiella

A de la pyridine refroidie à 0°C dans la glace, on ajoute de l'acide chlorosulfonique (0,4 cm³). Après retour à température ambiante, on ajoute le galactanne extrait de Klebsiella pneumoniae (25 mg) en suspension dans la pyridine (24 cm³). On chauffe le mélange pendant 3 heures à 80°C, refroidit à température ambiante, dilue avec de l'eau distillée (30 cm³) et finalement traite par une solution 2,5 M de soude (5 cm³). On concentre sous vide la solution obtenue, dialyse à l'eau distillée pendant 3 jours, congèle et lyophilise.

On obtient le dérivé sulfaté de galactanne extrait de Klebsiella ainsi recherché.

Dans le produit obtenu les hydroxyles sont sulfatés dans une proportion de 60%. Le poids moléculaire est voisin de 7000.

### Préparation du galactanne extrait de Klebsiella pneumoniae.

On solubilise 334 mg d'acylglycannes de Klebsiella (obtenus comme indiqué à l'exemple 1 de la demande française n° 2 574 429 à partir de la souche déposée à l'Institut Pasteur sous le n° I-103) dans 33,4 cm³ de solution d'acide acétique à 1% et l'on chauffe à 100°C pendant 90 minutes. Après refroidissement, on sépare le précipité contenant la fraction lipidique par centrifugation à 2000 g pendant 30 minutes. On lyophilise le surnageant et isole 200 mg de résidu que l'on redissout dans 4 cm³ d'eau. On chromatographie, par fraction de 1 cm3, sur une colonne pour chromatographie à haute performance d'échangeur d'anions Whatman Magnum 9 SAX (9,4 mm × 50 cm), en éluant par l'eau pendant 30 minutes, sous un débit de 2 cm³/minute en détectant à 200 nm. On isole la fraction renfermant les oses neutres repérés à 492 nm, après le test au phénol-sulfurique. On isole par lyophilisation 55 mg de fraction neutre.

— isolement du galactanne

Le lyophilisat précédent est dissous dans un tampon eau-acide acétique-pyridine (973-7-20), puis on soumet à une filtration sur gel par chromatographie sur colonne de Biogel A.1.5M (2 cm × 1.5 m), équilibrée avec le même tampon. On recueille la fraction détectée au test phénol-sulfurique. On stérilise par filtration sur membrane 0,22 micron, puis on lyophilise et obtient 22 mg du galactanne attendu.

### EXEMPLE 2 : Dérivé sulfaté du galactanne extrait de Klebsiella

A de la pyridine refroidie à 0°C dans de la glace, on ajoute de l'acide chlorosulfonique (4 cm³). Après retour à la température ambiante, on ajoute le galactanne extrait de Klebsiella pneumoniae (préparé comme indiqué dans la préparation donnée à l'exemple 1) (85 mg) en suspension dans la pyridine (24 cm³). On chauffe le mélange pendant 2 heures à 80°C, refroidit à température ambiante et dilue avec de l'eau distillée (30 cm³). On amène le pH du mélange réactionnel à 6 par addition d'une solution de soude 2,5 M. On dialyse à l'eau distillée la solution obtenue, congèle et lyophilise.

On obtient ainsi 32 mg de dérivé sulfaté de galactanne extrait de Klebsiella pneumoniae.

### EXEMPLE 3 :

On a préparé des comprimés répondant à la formule :
— Produit de l'exemple 1........................ 1 mg
— Excipient q.s pour un comprimé terminé à.... 100 mg
(détail de l'excipient : lactose, amidon, talc, stéarate de magnésium).

### EXEMPLE 4 :

On a préparé des comprimés répondant à la formule :
— Produit de l'exemple 2....................... 1 mg

— Excipient q.s pour un comprimé terminé à.... 100 mg
(détail de l'excipient : lactose, amidon, talc, stéarate de magnésium).

## EXEMPLE 5 :

On a préparé des aérosols délivrant des doses contenant chacune :
— Produit de l'exemple 1........................ 0,5 mg
— Emulsifiant.................................... 0,15 mg
— Propulseur.................................... 50,00 mg

## EXEMPLE 6 :

On a préparé une crème répondant à la formule :
— Produit de l'exemple 2........................... 1 mg
— Excipient : alcool 2-octyl-dodécanol, alcool
cétostéarylique, sulfate de sodium,
parahydro-xybenzoate de méthyle et de propyle,
eau purifiée q.s.p............................... 10 mg.

## Etude biochimique

L'activité du dérivé sulfaté du galactanne extrait de Klebsiella est déterminée par la mesure de l'inhibition des syncitia (Gruters et al Nature Vol 330 p 74 - 77,5, Novembre 1987) et par la détermination de l'activité protectrice exercée vis-à-vis de certaines cultures cellulaires.

## A) Preparation et titrage du virus

### 1°/ Préparation du virus

— surnageant des cellules H 9 III$_B$ (infectées de façon chronique par le HIV), cultivées pendant 48 heures à partir de $10^5$ cellules/cm$^3$
— surnageant centrifugé, filtré à 0,45 micronmole, aliquoté et stocké à -80°C.

### 2°/ Titrage du virus

— reverse transcriptase (RT)

Sur le surnageant frais $4,0.10^6$ cpm/cm$^3$
Après décongélation $4,5.10^6$ cpm/cm$^3$
— test MT4/MTT
technique :
100 microlitres de dilutions en série de virus dans une plaque de microtitration
- addition de 100 microlitres de suspension cellulaire contenant $5.10^4$ cellules MT4
- culture 7 jours à 37°C, 5% $CO_2$
- addition du MTT (sel de tétrazolium colorant de viabilité)
- incubation 4 heures à 37°C.
- arrêt de la réaction par addition d'une solution d'acide chlorhydrique dans l'isopropane
- lecture de la densité optique (DO) à 540 nm (après dissolution des cristaux).

## Résultats

On détermine le rapport de la densité optique des cellules infectées (cytotoxicité virale = DO faible) sur densité optique des cellules infectées (viabilité et DO maximales) en fonction de la dilution de virus (moyenne sur 3 puits).
Plus les cellules sont infectées et plus ce rapport est faible.

## B) Activité protectrice du dérivé sulfaté du galactanne extrait de Klebsiella

**1°/ Inhibition des Syncitia**

On effectue une coculture des cellules H 9 III (infectées de façon chronique $10^5$ cellules/cm³) avec les cellules SUP T1 (non infectées : $2 \times 10^5$ cellules/cm³).

Il y a formation de syncitia que l'on compte au microscope.

Chaque type de cellules est ou non préincubé avec le produit étudié, une nuit à 37°C.

S'il n'y a pas de préincubation, le produit étudié est ajouté sur les cellules au moment où l'on effectue la culture.

Lorsqu'il y a inhibition de la formation de Syncitia dans les cellules traitées par rapport aux cellules témoins, le produit étudié est protecteur.

Les résultats obtenus figurent ci-après :

### TABLEAU 1

**coculture avec le produit étudié sans incubation préalable**

| Produit Exemple 1 en microgrammes/cm³ | Pourcentage d'inhibition essai N°1 | essai N° 2 |
|---|---|---|
| 1 | 0 | 10 |
| 10 | 42 | 17 |
| 100 | 100 | 100 |

### TABLEAU 2

**préincubation avec le produit étudié 1 nuit à 37°C avant la coculture**

| Produit Exemple 1 en microgrammes/cm³ | Pourcentage d'inhibition cellules H 9 III | cellules Sup Ti |
|---|---|---|
| 1 | 17 | 23 |
| 10 | 23 | 27 |
| 100 | 100 | 100 |

**2°/ Test MT4/MTT**

On utilise des cellules MT4 à $10^6$ cellules/cm³. On dilue au 1/2 avec ou sans le produit étudié à différentes concentrations. On introduit sur une plaque de microtitration 100 microlitres de virus HIV à différentes dilutions et 100 microlitres de suspension cellulaire prétraitée.

On incube à 37°C pendant 7 jours, ajoute le MTT et effectue une lecture au spectrophotomètre.
On détermine le pourcentage d'inhibition de l'infection des cellules MT4.
Les résultats obtenus figurent ci-après :

### TABLEAU 3

| Produit Exemple 1 en microgrammes/cm$^3$ | Pourcentage d'inhibition |
|---|---|
| 1 | 74 |
| 10 | 99 |
| 100 | 84 |

### 3°/ Test H 9

On utilise des cellules H9 que l'on traite au polybrène, lave, reprend dans 100 microlitres de milieu, avec ou sans produit étudié à différentes concentrations.

On incube 2 Heures à 37°C ajoute 100 microlitres de virus HIV au 1/10, maintient 1 heure à 37°C, lave.

On reprend les cellules dans 1 cm³ de milieu avec ou sans produit étudié, répartit en 2 puits 500 microlitres par puits et 2. × 10⁵ cellules par puits.

A J + 4, on ajoute 500 microlitres de milieu de culture avec ou sans produit étudié.

A J + 8, on remplace la moitié du surnageant par du milieu neuf.

A J + 11, on effectue un dosage de reverse transcriptase sur le surnageant et une détermination immunofluorescente sur les cellules.

Les résultats obtenus figurent ci-après :

TABLEAU 4

| | Reverse Transcriptase | | Immunofluorescence | |
|---|---|---|---|---|
| | $cpm/cm^3$ X $10^4$ | %Inhibition | % cellules fluores- centes | % Inhi- bition |
| Cellules non infectées | 1,68 | | 0 | |
| Cellules infectées | 291 | | 51 | |
| Produit exemple 1 (concentration en microgram- mes/$cm^3$ | | | | |
| 1 | 6,42 | 76 | 3 | 94 |
| 10 | 1,16 | 100 | 0 | 100 |
| 100 | 1,12 | 100 | 0 | 100 |

Les résultats obtenus dans les différents tests montrent que le produit étudié inhibe de manière très importante la formation des syncitia et qu'il protège les cellules étudiées de l'infection causée par le virus HIV.

## Etude de l'activité anti-élastase.

L'activité anti-élastase a été déterminée par dosage spectrophotométrique vis à vis de l'élastase leucocytaire humaine (par une technique analogue à celle décrite par Boudier et al. Clin. Chim. Acta. 132, 309-315 (1983).

| Produit de l'exemple | Elastase leucocytaire humaine |
|---|---|
| 1 | Ki : 1 x $10^{-8}$M |

## Revendications

1. Nouveau dérivé sulfaté du galactanne extrait de Klebsiella, caractérisé en ce qu'il est essentiellement constitué d'oses neutres sulfatés dans une proportion de 20 à 90% des hydroxyles.

2. Dérivé sulfaté du galactanne selon la revendication 1, caractérisé en ce qu'il est essentiellement constitué d'oses neutres sulfatés dans une proportion de 40 à 80% des hydroxyles.

3. Dérivé sulfaté du galactanne selon la revendication 1 ou 2, caractérisé en ce qu'il est essentiellement constitué d'oses neutres sulfatés dans une proportion de 50 à 70% des hydroxyles.

4. Dérivé sulfaté du galactanne selon l'une quelconque des revendications 1, 2 ou 3, caractérisé en ce qu'il est constitué d'un enchaînement linéaire de galactose en 1-3 et a un poids moléculaire compris entre 5000 et 12000.

5. Dérivé sulfaté du galactanne selon l'une quelconque des revendications 1, 2, 3 ou 4, caractérisé en ce qu'il provient de Klebsiella pneumoniae.

6. Dérivé sulfaté du galactanne selon la revendication 5, caractérisé en ce qu'il provient de la souche déposée à l'Institut Pasteur à Paris ou Collection Nationale de Culture de Microorganismes (CNCM) sous les numéros 52145 et 1-163 et à la National Culture Type Collection sous le n° 5055.

7. Procédé de préparation du nouveau dérivé sulfaté du galactanne extrait de Klebsiella tel que défini à l'une quelconque des revendications 1 à 6 qui consiste à traiter un acylglycanne extrait de Klebsiella, essentiellement constitué d'environ 80% d'oses neutres, 20% de lipides, moins de 2% de protéines et ayant un poids moléculaire d'environ 12500, par hydrolyse acide douce, chromatographie à haute performance sur échangeur d'anions, récupère la fraction non retenue, soumet à une filtration sur gel, récupère la fraction contenant le galactanne de poids moléculaire compris entre 4000 et 10000, procédé caractérisé en ce que l'on sulfate la fraction de galactanne ainsi obtenu, purifie par dialyse et isole le dérivé sulfaté du galactanne ainsi obtenu.

8. Procédé selon la revendication 7, caractérisé en ce que la sulfatation de la fraction de galactanne est effectuée au moyen d'un acide sulfonique tel que l'acide chlorosulfonique au sein d'une amine telle que la pyridine ou au moyen d'un sulfonate tel que le N-sulfonate de pipéridine en opérant par chauffage du milieu réactionnel et la dialyse est effectuée contre de l'eau distillée.

9. Le dérivé sulfaté du galactanne tel qu'obtenu par le procédé défini à l'une quelconque des revendications 7 ou 8.

10. Médicaments, caractérisés en ce qu'ils sont constitués par les dérivés sulfatés du galactanne tels que définis à l'une quelconque des revendications 1 à 6.

11. Médicament, caractérisé en ce qu'il est constitué par le dérivé sulfaté du galactanne tel qUe défini à la revendication 9.

12. Compositions pharmaceutiques, caractérisées en ce qu'elles renferment, à titre de principe actif, l'un au moins des médicaments tels que définis à l'une quelconque des revendications 10 ou 11.

## Revendications pour les Etats contractants suivants : ES, GR

1. Procédé pour préparer un nouveau dérivé sulfaté du galactanne extrait de Klebsiella, caractérisé en ce qu'il est essentiellement constitué d'oses neutres sulfatés dans une proportion de 20 à 90% des hydroxyles, caractérisé en ce que l'on traite un acylglycanne extrait de Klebsiella, essentiellement constitué d'environ 80% d'oses neutres, 20% de lipides, moins de 2% de protéines et ayant un poids moléculaire d'environ 12500, par hydrolyse acide douce, chromatographie à haute performance sur échangeur d'anions, récupère la fraction non retenue, soumet à une filtration sur gel, récupère la fraction contenant le galactanne de poids moléculaire compris entre 4000 et 10000, procédé caractérisé en ce que l'on sulfate la fraction de galactanne ainsi obtenu, purifie par dialyse et isole le dérivé sulfaté du galactanne ainsi obtenu.

2. Procédé selon la revendication 1, caractérisé en ce que la sulfatation de la fraction de galactanne est effectuée au moyen d'un acide sulfonique tel que l'acide chlorosulfonique au sein d'une amine telle que la pyridine ou au moyen d'un sulfonate tel que le N-sulfonate de pipéridine en opérant par chauffage du milieu réactionnel et la dialyse est effectuée contre de l'eau distillée.

3. Procédé selon la revendication 1 ou 2, caractérisé en ce que l'on sulfate 40 à 80% des hydroxyles.

4. Procédé selon la revendication 1, 2 ou 3, caractérisé en ce que l'on sulfate 50 à 70% des hydroxyles.

5. Procédé selon l'une quelconque des revendications 1 à 4, caractérisé en ce que l'on sulfate un galactanne choisi de manière telle que l'on obtienne un dérivé sulfaté du galactanne constitué d'un enchaînement linéaire de galactose en 1-3 et a un poids moléculaire compris entre 5000 et 12000.

6. Procédé selon l'une quelconque des revendications 1 à 5, caractérisé en ce que l'on utilise au départ un acylglycanne extrait de Klebsiella pneumoniae.

7. Procédé selon la revendication 6, caractérisé en ce que l'on utilise au départ un acylglycanne provenant de la souche déposée à l'Institut Pasteur à Paris ou Collection Nationale de Culture de Microorganismes (CNCM) sous les numéros 52145 et 1-163 et à la National Culture Type Collection sous le n° 5055.

8. Procédé de préparation de compositions pharmaceutiques caractérisé en ce que l'on met à titre de principe actif l'un au moins des dérivés sulfatés du galactanne tels que définis à la revendication 1 sous une forme destinée à cet usage.